# EUROPEAN PATENT APPLICATION

(11) **EP 0 974 581 A1**
(43) Date of publication of application: **26.01.2000**
(21) Application number: 98941863.7
(22) Date of filing: 14.09.1998
(51) Int. Cl.: C07C 409/24, C07C 407/00, C07D 313/04

(54) **PROCESS FOR PREPARING EQUILIBRIUM PEROXY ACID AND PROCESS FOR PRODUCING LACTONE**

(30) Priority: 16.09.1997 JP 26935497; 19.02.1998 JP 5435898
(71) Applicant: DAICEL CHEMICAL INDUSTRIES, Ltd., Sakai-shi, Osaka 590-0905 (JP)
(72) Inventor: MATSUDA, Hirokazu, Hiroshima 739-0651 (JP); TAKEMOTO, Etsuo, Hiroshima 739-0421 (JP)
(74) Representative: Portal, Gérard
(86) International application number: JP9804135
(87) International publication number: WO9914190

(57) **Abstract**

The present invention relates to a method of producing an equilibrium peracid mixture containing not more than 1.5% by weight of water and not more than 1.5% by weight of hydrogen peroxide by reacting hydrogen peroxide and carboxylic acid(s) in a reaction-distillation apparatus while removing water. The present invention also relates to the reaction between the above-mentioned equilibrium peracid mixture and a cyclic ketone. According to the method of the present invention, the selectivity to the lactone can be increased without any decrease of the initial reactivity in the conversion of the cyclic ketone.

## Description

### TECHNICAL FIELD

The present invention relates to a method for the preparation of an equilibrium peracid mixture and to a method for preparing a lactone by using this equilibrium peracid mixture.

More particularly, the present invention relates to a method for preparing an equilibrium peracid mixture which contains not more than 1.5% by weight of water and not more than 1.5% by weight of hydrogen peroxide, by reacting hydrogen peroxide and carboxylic acid(s) in a reaction-distillation apparatus while removing water.

The present invention also relates to a method for producing a lactone by reacting a cyclic ketone with the above-mentioned equilibrium peracid mixture in a reaction-distillation apparatus while removing water. According to the method for preparing a lactone of the present invention, the initial reactivity in the conversion from a cyclic ketone to a lactone and the selectivity to the lactone are improved.

### TECHNICAL BACKGROUND

Among the lactones, for example, ε-caprolactone is a compound used as a starting material for polymers like polycaprolactones which are used as forming materials and polyester polyols which are used for polyurethanes. As a typical method for producing ε-caprolactone, a method consisting of oxidising cyclohexane is already known. This method of oxidising cyclohexane consists in using an organic peracid such as peracetic acid, as an oxidising agent.

The peracids having a high concentration are not stable and are thus highly dangerous. When using a peracid in a reaction, this peracid is often used in the form of an equilibrium peracid mixture. Equilibrium peracid mixture means here a chemically equilibrated mixture of hydrogen peroxide, carboxylic acid(s) and peracid, hydrogen peroxide and carboxylic acid(s) being the starting material to obtain the peracid.

A commonly known method of producing equilibrium peracid mixtures is to synthesise peracid from hydrogen peroxide and carboxylic acid(s).

However, when using an equilibrium peracid as an oxidising agent in a reaction, the selectivity of the reaction product decreases because of water and hydrogen peroxide which are contained in the equilibrium peracid mixture. For example, when synthesising a lactone by oxidising a cyclic ketone, the water and the hydrogen peroxide contained in the equilibrium peracid mixture are factors which reduce the initial reactivity in the conversion of the cyclic ketone to a lactone and the selectivity thereof.

On the other hand, it is known to synthesise higher concentration peracid through using hydrogen peroxide and carboxylic acid in higher concentration. For example, according to Kirk-Othmer Encyclopedia of Chem. Tech. Ist, Suppl. P. 622 (1957), it is possible to obtain an equilibrium mixture containing 45% by weight of peracetic acid, 35% by weight of acetic acid, 6% by weight of hydrogen peroxide and 14% by weight of water from a mixture containing 90% by weight of hydrogen peroxide and an amount by mole of acetic acid equivalent to 1.5 times of the amount of hydrogen peroxide. It has also been reported that it is possible to obtain an equilibrium mixture containing 23% by weight of peracetic acid, 45% by weight of acetic acid, 7% by weight or hydrogen peroxide and 25% by weight of water by reacting a mixture containing 50% by weight of hydrogen peroxide and a molar amount acetic acid corresponding to 2 times of the molar amount of hydrogen peroxide.

According to these examples, when reacting hydrogen peroxide and carboxylic acid(s) in higher concentration, hydrogen peroxide which has not reacted, water and synthesised peracids remain at the equilibrium ; water and synthesised peracid(s) being present in the same molar amount. For this reason, even when using hydrogen peroxide having a concentration of not less than 50% by weight, as a starting material, not less than 10% by weight of water and not less than 5% by weight of hydrogen peroxide still remain in the equilibrium peracid mixture.

Further, a method which consists in reacting acetic acid and hydrogen peroxide in the presence of sulphuric acid catalyst has been reported in Ullman's Enzyklopadie der technischen Chemie, 3^{rd} edition, 13^{th} vol. p. 254. According to this method, a high concentration in peracetic acid of 40-42% by weight is obtained. However, the amount of water and hydrogen peroxide is 10-14% by weight and 4-6% by weight respectively.
Moreover, a method of producing an equilibrium diluted solution from a diluted solution of lower aliphatic per acids and hydrogen peroxide is reported in JP-A-05-504357. According to this method, a reaction mixture which is rich in lower aliphatic peracids can be quickly obtained by bringing hydrogen peroxide and lower aliphatic acids into contact in an aqueous reaction mixture. In this reaction, the concentration of hydrogen peroxide and low-order chain-acids are as high as 15-30% by weight and 27-70% by weight, respectively. This reaction mixture is then diluted with water and a suitable amount of lower aliphatic acids and/or hydrogen peroxide so as to form a diluted solution which is in an equilibrium mixture. According to this method, the concentration of water and hydrogen peroxide are 45% and 27% respectively in the reaction mixture. Since this mixture is diluted to form a dilute solution which is at equilibrium, the concentrations in hydrogen peroxide and water are still higher.

A method for producing peracetic acid by reacting, in an organic solvent, hydrogen peroxide and acetic acid under a sulphuric acid catalysis in a distillation apparatus is disclosed in JP-A-64016760.

JP-A-06-340617 discloses a method of producing a composition of peracetic acid which does not correspond to a harmful product according to the Fire-Defence Law. This composition is obtained by mixing, in suitable amounts, hydrogen peroxide, acetic acid and water. However, also in this case, the final peracetic acid mixture contains water in 50% by weight or more and hydrogen peroxide in 7% by weight or more.

In view of the existing circumstances, the purpose of the present invention is to provide a method for obtaining an equilibrium peracid mixture which contains small amounts of water and hydrogen peroxide from a mixture of carboxylic acid and hydrogen peroxide and also to provide a method for oxidising a cyclic ketone to the corresponding lactone by using this equilibrium peracid mixture, as an oxidising agent. The use of such an equilibrium peracid mixture improves the initial reactivity in the conversion of a cyclic ketone to a lactone and the selectivity thereof.

### DISCLOSURE OF THE INVENTION

The inventors of the present invention have performed an intensive research concerning the methods of removing water and hydrogen peroxide from an equilibrium peracid mixture. The result of such a research is that it is possible to obtain an equilibrium peracid mixture containing not more than 1.5% by weight of water and not more than 1.5% by weight of hydrogen peroxide by using a distillation apparatus and by removing water during reaction. A second result of this research is that it is possible to convert a cyclic ketone to a lactone by using such an equilibrium peracid mixture containing a small amount of water and hydrogen peroxide. The use of such an equilibrium mixture improves the initial reactivity in the conversion of a cyclic ketone to a lactone and the selectivity thereof.
The present invention is as follows :
1) A method of producing an equilibrium peracid mixture from reacting hydrogen peroxide and carboxylic acid(s) wherein water is removed during reaction by using a reaction-distillation apparatus.
2) The method described according to 1), wherein the reaction-distillation apparatus is equipped with a distillation column.
3) The method described according to 1), wherein the reaction-distillation apparatus is equipped with a distillation column having a theoretical-plates number ranging from 10 to 30.
4) The method as described in 1), 2) or 3), wherein the reaction is carried out while removing water until the water concentration becomes equal to or less than 1.5% by weight.
5) The method as described in 1), 2), 3) or 4), wherein the reaction is carried out while removing water until the hydrogen peroxide concentration becomes equal to or less than 1.5% by weight.
6) The method as described in 1), 2), 3), 4), or 5) wherein the carboxylic acid(s) is (are) selected from at least one of formic acid, acetic acid, propionic acid, trifluoroacetic acid, butyric acid and/or benzoic acid.
7) A method of forming a lactone by reacting an equilibrium peracid mixture and a cyclic ketone wherein the equilibrium peracid mixture is obtained by any one of the methods described in 1), 2), 3), 4), 5) or 6).
8) The method for forming a lactone as described in 7), wherein the cyclic ketone is a monoketone having a saturated carbon ring of 3-12 carbon atoms.

According to the present invention, it is possible to obtain an equilibrium peracid mixture containing a small amount of water and a small amount of hydrogen peroxide.

The advantage of the equilibrium peracid mixture obtained according to the present invention is that when it is used as an oxidising agent for the synthesis of a lactone, since it contains a small amount of water and hydrogen peroxide, the initial reactivity in the conversion of a cyclic ketone to a lactone and the selectivity thereof are improved compared to the prior art

### BEST MODE FOR CARRYING OUT THE INVENTION

In order to obtain the equilibrium peracid mixture of the present invention, a reaction-distillation apparatus must be used. When using a distillation apparatus, it is possible during the formation of an equilibrium peracid from hydrogen peroxide and carboxylic acid, at the step of the formation of the peracid to remove water before the acid reaches the state of an equilibrium peracid. Therefore it is possible to restrict the concentration of water and hydrogen peroxide to a lower concentration of 1.5% by weight or less.

According to a particularly advantageous characteristic of the present invention, it is possible to use common solutions of hydrogen peroxide having concentration of 35-65% by weight. Such solutions are available on the market. For example, an aqueous solution having a concentration of approximately 35% by weight, approximately 50% by weight, approximately 60% by weight or the like of hydrogen peroxide may be used. Particularly, solutions having a concentration of hydrogen peroxide of 50-60% by weight are preferably used. For example, an aqueous solution having a concentration of approximately 60% by weight of hydrogen peroxide is preferably used.

The carboxylic acids used in the present invention are formic acid, acetic acid, propionic acid, trifluoroacetic acid, butyric acid, isobutyric acid, benzoic acid or the like. Preferably, ethanoic acid and propionic acid are used. According to the invention, these carboxylic acids maybe used alone or in a mixture containing two or more acids.

The molar ratio of the hydrogen peroxide and the carboxylic acid in the mixture is : hydrogen peroxide/carboxylic acid = 0.05/1.5, preferably, 0.1/1.0 and more preferably, 0.1/0.5. When the amount of hydrogen peroxide is less than 0.05 mole, there is no production of equilibrium peracid because of a lack of hydrogen peroxide. When the amount of hydrogen peroxide is higher than 1.5 mole, it remains until the end of the reaction and the concentration in hydrogen peroxide cannot be more than 1.5% by weight in the final solution.

According to the present invention, in the case of obtaining the equilibrium peracid mixture, compounds for removing water by evaporation (hereinafter referred as entrainers) may be used. These entrainers are, for example, ethyl formate, ethyl acetate, ethyl proprionate, ethyl trifluoroethylate, ethyl butyrate, n-hexane, cyclohexane, ethyl benzene, 1,4-dioxane, toluene or the like. Preferably, ethyl acetate, ethyl propionate, n-hexane, cyclohexane, ethyl benzene and the like are used.

The molar amount of these compounds to be used is 0.1-20 times based on the molar amount of carboxylic acid, preferably 0.2-10 times based on the molar amount of the carboxylic acid and more preferably 0.3-5 times based on the molar amount of carboxylic acid. When this amount is less than 0.1 times based on the molar amount of carboxylic acid, water cannot be sufficiently removed. When this amount exceeds 20 times based on the molar amount of carboxylic acid, too large a distillation apparatus is required.

According to the invention, it is possible to use a catalyst with the hydrogen peroxide and the carboxylic acid to obtain the equilibrium peracid mixture.

Mineral acids such as sulphuric acid, phosphoric acid or the like, paratoluenesulphonic acid and an ion-exchange resin can be used as catalysts. Preferably, the ion-exchange resin are used but catalysts that can be used according to the invention are not limited to those. Strong acid ion-exchange resins, strong base ion-exchange resins, weak acid ion-exchange resins, weak base ion-exchange resins and chelate resins can be used for example. Preferably, the strong-acid ion-exchange resins are used.

The catalyst is used in an amount of 0.05-30% by weight of the total solution, preferably of 0.1-10% by weight and more preferably 0.1-3% by weight. When the amount of catalyst is less than 0.05% by weight, it takes no less than 7 hours to reach the equilibrium. When the amount of catalyst is more than 30% by weight, it becomes difficult to completely remove water with a falling-film evaporator (hereinafter referred as FFE).

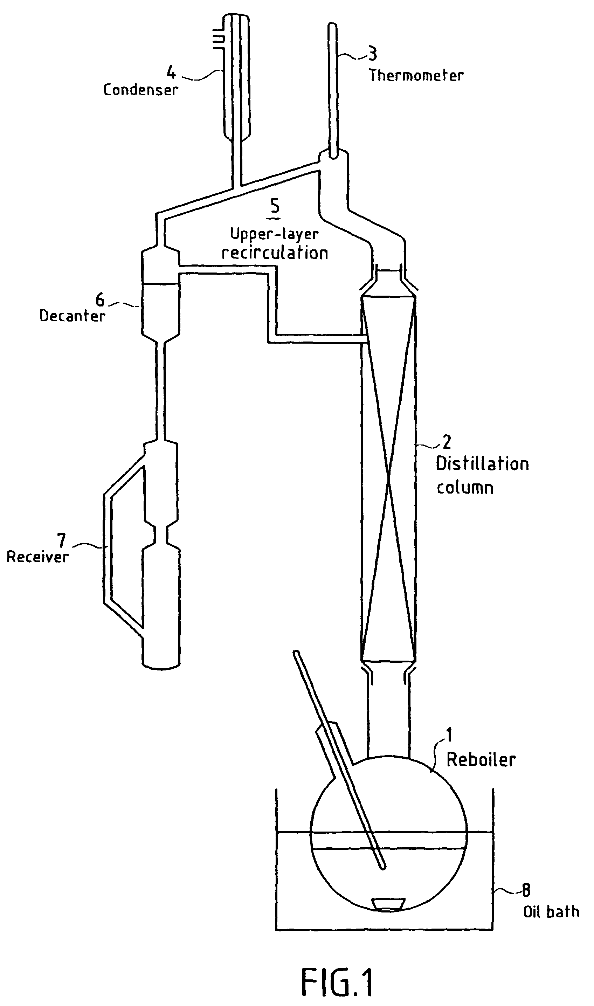

Figure 1 shows an example of a reaction-distillation apparatus that may be used according to the present invention.

This reaction-distillation apparatus comprises a reactor (1 : reboiler), a distillation column set (2 : Distillation column, Oldershaw-type distillation apparatus) on the top portion of the reactor, a condenser 4 and optionally a liquid-liquid separator (6 : decanter).

Hydrogen peroxide, carboxylic acid and optionally the catalyst are introduced in the reactor. The distillation column may be a plate column or a packed column. Preferably, the number of theoretical plates is 10-30 stages.

The condenser is used to remove water which has been distilled out of the reaction system. The liquid-liquid extractor is used when an entrainer has been added ; the mixture of entrainer and water which boil together is condensed in the condenser and then introduced into a refluxing apparatus equipped with the liquid-liquid separator to separate water from the entrainer. The entrainer is then recycled to the top of the distillation column and/or the reactor. For example, an Oldershaw-type distillation apparatus may be used.

The reaction-distillation may be a batch distillation, a semi-batch distillation or a continuous distillation.

For example, it is possible to continuously discharge the equilibrium peracid obtained from a continuous distillation out of the reactor and then reacting it with a cyclic ketone to continuously produce a lactone.

The invention is characterised in that water is continuously removed during the reaction to obtain an equilibrium peracid mixture. There are methods in which water is removed after having obtained an equilibrium peracid mixture by introducing an entrainer and using an FFE or an Oldershaw-type distillation apparatus. However, according to these methods, it is not possible to produce an equilibrium peracid mixture containing not more than 1.5% by weight of water and not more than 1.5% by weight of hydrogen peroxide.

In order to obtain an equilibrium peracid mixture, the temperature of the bottom of the distillation column in the reaction-distillation apparatus is 30-100°C and preferably 40-80°C. When this temperature is lower than 30°C, it is not possible to obtain a sufficient distilled volume. When this temperature is higher than 100°C, the reaction is too fast and results in being dangerous. The temperature of the top of the column is 20°C-100°C and preferably controlled to 30°C-80°C. When this temperature is less than 20°C, the amount of water and hydrogen peroxide remaining inside the column is more than 1.5% by weight. When this temperature is higher than 100°C, the produced equilibrium peracid mixture is also distilled out. Regarding the pressure, the initial pressure inside the distillation apparatus is a quite low reduced-pressure such as a pressure of 600-670 torr. When the initial pressure is less than 600 torr, the equilibrium peracid mixture cannot be obtained because the reaction-distillation suddenly occurs. Further, when the pressure is more than 670 torr, the production of equilibrium peracid mixture is delayed. The pressure is finally 50-500 torr, preferably, 100-450 torr and more preferably controlled in the range of 150-400 torr. When the pressure is finally less than 50 torr, the equilibrium peracid mixture produced is distilled out ; when the final pressure is more than 500 torr, the amount of water and hydrogen peroxide remaining inside the column is more than 1.5% by weight. Under the above-mentioned conditions and through distilling water out during the reaction, it is possible to obtain such an equilibrium peracid mixture.

According to the present invention, the starting materials, catalyst and entrainer are preferably introduced according to the following order : (1) starting material → (2) catalyst → (3) entrainer → or (1) starting material → (2) entrainer → (3) catalyst.

According to the method of preparing a lactone of the present invention, an equilibrium peracid mixture containing not more than 1.5% by weight of water or not more than 1.5% by weight of hydrogen peroxide is preferably reacted with a cyclic ketone. In particular, an equilibrium peracid mixture containing not more than 1.5% by weight of water and not more than 1.5% by weight of hydrogen peroxide is preferably used. The selectivity of a cyclic ketone to a lactone is improved when such an equilibrium peracid mixture is used.

The molar ratio of the peracid and the cyclic ketone in the peracid equilibrium mixture is preferably as follows : peracid/ cyclic ketone = 0.1/2, preferably, 0.2/1.5 and more preferably 0.3/1.0. In these ranges, the selectivity of a cyclic ketone to a lactone can be improved.

The temperature of the reaction is 25-50°C, preferably 30-45°C and more preferably 32-41°C. When the temperature is lower than 25°C or higher than 50°C, the selectivity to a lactone is less than 70%. The reaction pressure is 0.5-2atm, preferably, 0.7-1.5atm and more preferably 0.8-1.2atm. When the reaction pressure is less than 0.5atm, the kinetics of the reaction (the reaction rate) is extremely delayed and when the reaction pressure is higher than 2 atm, the reaction becomes a high-pressure reaction and thus the apparatus costs become high.

It is possible to use a catalyst or a solvent for the reaction converting a cyclic ketone into a lactone using an equilibrium peracid mixture. N-hexane, cyclohexane, toluene, pseudocumene or the like may be used, for example, as solvents. Preferably, n-hexane or cyclohexane are used. The amount of solvent used depends on the volume capacity of the apparatus. An amount of solvent corresponding to 10-70% by weight and preferably 30-50% by weight with respect to the total weight amount of the cyclic ketone and equilibrium peracid used.

Sulphuric acid, paratoluenesulphonic acid, or other acid catalyst, or an ion exchange resin may be used as catalyst for this reaction.

Preferably, a saturated cyclic monoketone having a ring of 3-12 carbon atoms, such as, for example, cyclopropanone, cyclobutanone, cyclopentanone, cyclohexanone, cyclooctanone, cyclodecanone cyclododecanone or the like may be used.

The saturated cyclic monoketones may be substituted with an alkyl group having 1-3 carbon atoms. This alkyl group may be, for example, a methyl group, an ethyl group, a propyl group or an isopropyl group. The ring may also be substituted with a plurality of alkyl groups. The number of these alkyl groups is only limited by the number of carbon atoms in the ring. The same carbon atom of the ring may be substituted by one or two alkyl groups. Specifically, methyl cyclopropanone, methyl cyclobutanone such as 2-methyl-cyclobutanone, propyl cyclobutanone such as 2-propylcyclobutanone, methyl cycloheptanone such as 2-methylcycloheptanone, 2-methylcyclohexanone, 3-methylcyclohexanone, 4-methylcylohexanone, or further methyl cyclohexanone, methyl cyclooctanone such as 2-methylcyclooctanone, methyl cyclodecanone such as 2-methylcyclodecanone, methyl cyclododecanone such as 2-methylcyclododecanone may be given as non-limiting examples.

Further, the number of alkyl groups having 1-3 carbon atoms is not limited to any one group. According to the present invention, trimethyl derivatives of cyclohexanone, such as 3, 3, 5-trimethylcyclohexanone, are also preferably used. Moreover, in the case of a plurality of alkyl groups, these groups may also be different like in 3, 3-dimethyl-6-ethylcyclohexanone or the like.

According to the method of preparing a lactone of the present invention, the following lactones can be, for example, obtained from the following compounds : β-propiolactone may be obtained from cyclopropanone, γ-butyrolactone may be obtained from cyclobutanone, γ- valerolactone can be obtained from 2-methylcyclobutanone, γ-caprolactone can be obtained from 2-ethylcyclobutanone, γ-caprilolactone may be obtained from 2-propylcyclobutanone, γ-palmitolactone may be obtained from 2-undecylcyclobutanone, δ-valerolactone may be obtained from cyclopentanone, δ-caprolactone may be obtained from 2-methylcyclopentanone, ε-caprolactone may be obtained from cyclohexanone, ε-caprilolactone may be obtained from 2-ethylcyclohexanone and ε-laurolactone may be obtained from 2-hexylcyclohexanone.

### BRIEF DESCRIPTION OF DRAWINGS

Fig. 1 briefly shows an example of the reaction-distillation apparatus used to obtain an equilibrium peracid mixture from hydrogen peroxide and carboxylic acid.
- 1 :: reboiler
- 2 :: distillation column (Oldershaw-type)
- 3 :: thermometer (°C)
- 4 :: condenser
- 5 :: upper layer recirculation
- 6 :: decanter
- 7 :: receiver
- 8 :: oil bath

### EXAMPLES

Hereinafter, the present invention will be more specifically explained on the basis of the following examples. However, the present invention is not limited to these examples.

### Example 1

370g of propionic acid, 94.5g of an aqueous solution containing 60% by weight of hydrogen peroxide, 2.1g of a strong acid ion-exchange resin (trademark DOWEX-50W produced by DOW CO. Ltd;) and 490g of ethyl propionate used as an entrainer were introduced and mixed in a reaction-distillation apparatus equipped with a dstillation column-type distillation column having a diameter of 40 mm and 30 plates. The reaction-distillation was carried out for 5 hours with the temperature of the bottom of the column being 71-75°C, the temperature of the top of the column being 45-48°C and the pressure of the top of the column being 190-260 torr. The catalyst was then removed by using a falling-film evaporator (FFE) equipped with a fractionating-distillation column. The pressure of the top of this fractionating-distillation column was set at 50 torr and the temperature thereof at 72°C, the reflux ratio was 0.3. 620g of an equilibrium peracid mixture was then obtained. The equilibrium peracid mixture obtained was then analysed. The results of this analysis showed that the mixture obtained contained 18.8% by weight of perpropionic acid, 0.21% by weight of water and 0.23% by weight of hydrogen peroxide. The concentration of water and hydrogen peroxide were both very low.

### Example 2

186g of acetic acid, 58.9g of an aqueous solution containing 60% by weight of hydrogen peroxide, 1.2g of an ion-exchange resin and 156g of ethyl acetate used as an entrainer were introduced into a reaction-distillation apparatus and mixed together. The reaction distillation was performed according to Example 1 for 6 hours with the temperature of the bottom of the column being 64-67°C, the temperature of the top of the column being 41-45°C and the pressure of 250-300 ton. After removing the catalyst by using an FFE equipped with a fractionating-distillation column, 260g of an equilibrium peracid mixture were obtained. The results of the analysis of this mixture showed that its contained 17.6% by weight of peracetic acid, 1.4% by weight of water, 1.2% by weight of hydrogen peroxide. The water concentration and the hydrogen peroxide concentration were both not more than 1.5% by weight.

### Example 3 Except the amount of the ion exchange resin which was 6.0g (5 times the amount used in Example 2), 255g of an equilibrium peracid mixture were obtained under the same conditions as described in Example 2. The results of the analysis of this mixture showed that it contained 19.1% by weight of peracetic acid, 1.3% by weight of water, 1.2% by weight of hydrogen peroxide. The concentration in water and in hydrogen peroxide were both not more than 1.5% by weight

In the following Comparative Examples, in each case, the equilibrium peracid mixture which has been obtained was then dehydrated.

### Comparative Example 1

2039g of propionic acid, 526g of an aqueous solution containing 60% by weight of hydrogen peroxide and 15g of the ion-exchange resin used in Example 1 were introduced in a 3 litre flask and mixed for 5 days to produce an equilibrium perpropionic acid mixture. 173g of this mixture were distilled to remove water with 173g of n-hexane used as an entrainer. The temperature in the bottom of the distillation column was 61-64°C, the temperature in the top of the column was 44-45°C and the pressure thereof was 330-340 torr. 135g of an equilibrium perpropionic acid mixture were then obtained. The analysis of this equilibrium perpropionic acid mixture showed that it contained 7.9% by weight of water, 12.1% by weight of hydrogen peroxide and 17.1% by weight of perpropionic acid.

### Comparative Example 2

2781g of acetic acid, 526g of an aqueous solution containing 60% by weight of hydrogen peroxide and 15g of the same ion exchange resins as used in Example 1 were mixed in a 5 litre flask for 5 days to produce an equilibrium peracetic acid mixture. 172g of this mixture were introduced into a distillation column with 175g of ethyl acetate as an entrainer to remove water therefrom. 118g of an equilibrium peracetic acid mixture were then obtained. An analysis of this mixture showed that it contained a low water concentration of 1.2% by weight but the concentration in hydrogen peroxide was 3.3% by weight. The concentration of peracetic acid obtained was 15.2% by weight

The equilibrium peracid mixtures obtained in Examples 1 to 3 and in Comparative Examples 1 and 2 were reacted with cyclohexanone which is a cyclic ketone to produce ε-caprolactone.

### Example 4

The equilibrium peracid mixture obtained in Example 1 was used to form a mixture of an equilibrium peracid and cyclohexanone having a perpropionic acid/cyclohexanone molar ratio of 0.6/1.0. The reaction temperature was 37°C and the reaction pressure was 1 atm. After reacting for 1 hour, the conversion of the cyclohexane was 54.8% and the selectivity of cyclohexanone to ε-caprolactone was 91.7%.

### Example 5

The equilibrium peracid mixture obtained in Example 2 was used to form a mixture of an equilibrium peracid and cyclohexanone having a mole ratio peracetic acid/cyclohexanone molar ratio of 0.6/1.0. The reaction temperature was 37°C and the reaction pressure was 1atm. After reacting for one hour, the conversion of the cyclohexane was 55.6% and the selectivity of cyclohexanone to ε-caprolactone was 85.3%.

### Example 6

The equilibrium peracid mixture obtained in Example 3 was used to form a mixture of an equilibrium peracid and cyclohexanone having a peracetic acid/cyclohexanone molar ratio of 0.6/1.0. The reaction temperature was 37°C and the reaction pressure was 1atm. After reacting for 1 hour, the conversion of the cyclohexane was 57.2% and the selectivity of cyclohexanone to ε-caprolactone was 78.2%.

### Comparative Example 3

The equilibrium perpropionic acid mixture obtained in Comparative Example 1 was used to form a mixture of equilibrium peracid and cyclohexanone having a perpropionic acid/cyclohexanone molar ratio of 0.6/1.0. The reaction temperature was 37°C and the reaction pressure was 1atm. After reacting for 1 hour, the conversion of the cyclohexane was 53.2% and the selectivity of cyclohexanone to ε-caprolactone was 75.4%.

### Comparative Example 4

The equilibrium peracid mixture obtained in Comparative Example 2 was used to form a mixture of an equilibrium peracid and cyclohexanone having a peracetic acid/cyclohexanone molar ratio of 0.6/1.0. The reaction temperature was 37°C and the reaction pressure was 1atm. After reacting for 1 hour, the conversion of the cyclohexane was 53.5% and the selectivity of cyclohexanone to ε-caprolactone was 68.7%.

On the basis of the above-mentioned results, the peracid equilibrium mixtures obtained in Examples 1 to 3 and the peracid equilibrium mixtures obtained in Comparative Examples 1 and 2 can be compared. It is proved from such a comparison that the initial reactivity in the conversion of cyclohexanone to ε-caprolactone in Examples 1-3 is greatly improved as well as the selectivity thereof.

### INDUSTRIAL APPLICABILITY

The method of the present invention enables producing a peracid equilibrium mixture containing less than 1.5% by weight of water and less than 1.5% by weight of hydrogen peroxide by reacting hydrogen peroxide and (a) carboxylic acid(s) in a reaction-distillation apparatus while removing water during the reaction. The equilibrium peracid mixture obtained by reacting hydrogen peroxide and (a) carboxylic acid(s) in a reaction-distillation apparatus while removing water may be used, according to the present invention, as an oxidising agent to oxidise a cyclic ketone. Since the water content and the hydrogen peroxide content of such an equilibrium peracid mixture is low, the initial reactivity in the conversion of a cyclic ketone to a lactone and the selectivity thereof are improved.

According to the preparation method of the present invention, the carboxylic acid synthesised during the reaction of the ring lactone can be recycled for the preparation of the equilibrium peracid mixture itself. Consequently, a carboxylic acid is not a by-product. Further, since the carboxylic peracid is produced on the same production site, there is no location limitation due to the transport of the carboxylic peracid which is legally restricted.

## Claims

1. A method of preparing an equilibrium peracid mixture from hydrogen peroxide and carboxylic acid(s), characterised in that water is removed during reaction by using a reaction-distillation apparatus.

2. The method of claim 1, wherein said reaction-distillation apparatus is equipped with a distillation column.

3. The method of claim 1 or 2, wherein said reaction-distillation apparatus is equipped with a distillation column having a theoretical-plates number ranging from 10 to 30.

4. The method according to any one of claims 1 to 3, wherein the reaction is carried out while removing water until the water concentration becomes equal to or less than 1.5% by weight.

5. The method according to any one of claims 1 to 4, wherein the reaction is carried out while removing water until the hydrogen peroxide concentration becomes equal to or less than 1.5% by weight.

6. The method according to any one of claims 1 to 5, wherein said carboxylic acid is selected from at least one of formic acid, acetic acid, propionic acid, trifluoroacetic acid, butyric acid and benzoic acid.

7. A method of forming a lactone by reacting an equilibrium peracid mixture and a cyclic ketone wherein said equilibrium peracid mixture is obtained according to the method of any one of the claims 1 to 6.

8. The method for forming a lactone of claim 7, wherein said cyclic ketone is a monoketone having a saturated carbon ring of 3-12 carbon atoms.
